(19) **Europäisches Patentamt / European Patent Office / Office européen des brevets**

(11) **EP 4 628 054 A1**

(12) # EUROPEAN PATENT APPLICATION

(43) Date of publication:
**08.10.2025   Bulletin 2025/41**

(21) Application number: **24168372.1**

(22) Date of filing: **04.04.2024**

(51) International Patent Classification (IPC):
*A61F 2/58* (2006.01)      *A61F 2/72* (2006.01)
*A61F 5/01* (2006.01)      *B25J 9/00* (2006.01)
*A61F 2/70* (2006.01)

(52) Cooperative Patent Classification (CPC):
**A61F 2/583; A61F 2/72; A61F 5/013; B25J 9/0006;**
A61F 2002/701; A61F 2002/704; A61F 2005/0155

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Friedrich-Alexander-Universität
Erlangen-Nürnberg
91054 Erlangen (DE)**

(72) Inventors:
• **BRAUN, Dominik
  91058 Erlangen (DE)**

• **WALTER, Jonas
  91058 Erlangen (DE)**
• **DEL VECCHIO, Alessandro
  91052 Erlangen (DE)**
• **FRANKE, Jörg
  91080 Marolffstein (DE)**
• **SIMPETRU, Raul
  91080 Marloffstein (DE)**
• **WEBER, Nico
  91052 Erlangen (DE)**

(74) Representative: **Vossius & Partner
Patentanwälte Rechtsanwälte mbB
Siebertstraße 3
81675 München (DE)**

(54) **SYSTEM AND METHOD FOR CONTROLLING AN ASSISTING DEVICE**

(57)    The present disclosure relates to a system for controlling an assisting device for supporting or moving a patient's body part. The system comprises the assisting device; at least one actuator connected to the assisting device; at least one processor; at least one sensor configured to acquire a surface electromyographic, sEMG, signal on a user's skin. The processor is configured to determine an intended movement of the assisting device based on the sEMG signal and using an artificial intelligence model, and the processor is configured to control the at least one actuator according to the intended movement.

**Fig. 1**

EP 4 628 054 A1

**Description**

The present disclosure relates to a system and method for controlling an assisting device, for example an assisting device for supporting or moving a patient's body part. In particular, the present disclosure relates to a system and method to read injured muscle signals to automatically decode a patient's intent.

Surface electromyography (sEMG) is a non-invasive technique that records the electrical signals generated by muscles through electrodes placed on the skin. sEMG is the state-of-the-art method used to control active upper limb prostheses because of the association between its amplitude and the neural drive sent from the spinal cord to muscles.

However, accurately estimating the dynamics of a freely moving human hand using sEMG from extrinsic hand muscles is challenging. The present disclosure is concerned with mapping raw sEMG signals into dynamics or kinematics and evaluating a number of EMG signals and the type of pre-processing for enhancing performance of such a system. Moreover, the disclosure is concerned with improving a system for controlling an assisting device for supporting or moving a patient's body part by determining an intended movement of a patient.

The invention is specified by the independent claims. Preferred embodiments are defined in the dependent claims. In the following description, although numerous features may be designated as optional, it is nevertheless acknowledged that all features comprised in the independent claims are not to be read as optional.

The present disclosure relates to a system for controlling an assisting device for supporting or moving a patient's body part. The system comprises the assisting device; at least one actuator connected to the assisting device; at least one processor; at least one sensor configured to acquire a surface electromyographic, sEMG, signal on a user's skin. The processor is configured to determine an intended movement of the assisting device based on the sEMG signal and using an artificial intelligence model, and the processor is configured to control the at least one actuator according to the intended movement.

Various embodiments may preferably implement the following features.

The artificial intelligence model may be a machine learning model.

Preferably, the processor is configured to train the artificial intelligence model based on the sEMG signal.

Preferably, the assisting device is an orthosis, a prothesis or a robotic limb. Preferably, the orthosis is a hand orthosis.

Preferably, the assisting device is configured to independently move, via the actuators, the assisting device in at least two degrees of freedom. Preferably, the assisting device may be configured to independently move, via the actuators, in three degrees of freedom. One degree of freedom may be a digit or a gesture. Also, the degrees of freedom may be a thumb, and/or an index finger and/or at least one further finger.

Preferably, the assisting device further comprises at least one orientation sensor and/or at least one force sensor.

Preferably, the processor is further configured to apply at least one filter to the sEMG signal.

Preferably, the filter is a temporal filter, more preferably a Butterworth filter. Preferably, the filter is a spatial filter.

Preferably, the system comprises a plurality of sensors, wherein the processor is configured to determine an average of the sEMG signals of at least two of the plurality of sensors.

The disclosure further relates to a method for training an artificial intelligence model for controlling an assisting device for supporting or moving a patient's body part, the method comprising acquiring, via at least one sensor, a surface electromyographic, sEMG, signal on a user's skin; determining an intended movement of the assisting device based on the sEMG signal and the artificial intelligence model, and controlling at least one actuator of the assisting device according to the intended movement. The method further comprises training the artificial intelligence model based on the sEMG signal.

Various embodiments may preferably implement the following features.

The artificial intelligence model may be a machine learning model.

Preferably, the method further comprises applying at least one filter to the sEMG signal, wherein the filter preferably is a temporal filter, more preferably a Butterworth filter, and/or wherein the filter preferably is a spatial filter.

Preferably, the method further comprises determining an average of the sEMG signals of at least two of a plurality of sensors.

The present disclosure further relates to a corresponding method for controlling an assisting device for supporting or moving a patient's body part. The method comprises acquiring, by at least one sensor, a surface electromyographic, sEMG, signal on a user's skin, determining an intended movement of the assisting device based on the sEMG signal and using an artificial intelligence model and controlling at least one actuator of the assisting device according to the intended movement.

The invention is further described with reference to the figures. Therein, the same or similar elements are denoted by the same reference numerals.

Fig. 1 shows a schematic overview of the system according to an embodiment of the disclosure.

Fig. 2 shows a flow chart of an embodiment according to the present disclosure.

Fig. 3 shows a flow chart of an embodiment according to the present disclosure.

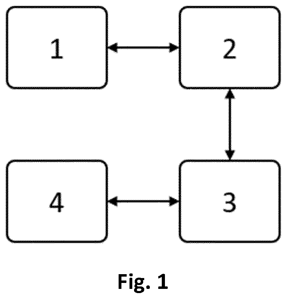

The system as described herein and as shown in Fig. 1 comprises an assisting device 1. The assisting device 1 may be configured to support or move a patient's body part, e.g., a limb. The assisting device 1 may be an orthosis, in particular a hand orthosis, a prothesis or a robotic limb.

At least one actuator 2 is connected to the assisting device 1. The actuator 2 may be a motor or the like and may be configured to control at least one degree of freedom of the assisting device 1. The actuator 2 may be mechanically connected to the assisting device 1 via wires, rods or the like.

At least one processor 3 may be provided and at least one sensor 4 configured to acquire or measure a surface electromyographic, sEMG, signal on a user's skin. Depending on the use case, the at least one sensor 4 can be placed on a certain area of the patient, e.g., an arm or a leg, to collect nerval signals of the patient via sEMG. The at least one sensor 4 may also be referred to as a non-invasive brain-machine interface (BMI).

The processor 3 is configured to determine an intended movement of the assisting device 1 based on the sEMG signal and using an artificial intelligence model. The processor 3 is further configured to control the at least one actuator 2 according to the intended movement. The artificial intelligence model may be a machine learning model.

A number of sensors 4 (electrodes) may be chosen according to the intended use. In an embodiment, the number of sensors 4 is between 1 and a few thousand, preferably between 1 and 2000, more preferably between 1 and 1000, and more preferably between 25 and 350. For exemplary purposes of the present disclosure, the sensors 4 may be positioned on a patient's forearm and/or wrist to control a hand orthosis. The sensors 4 may be grouped into matrices. The matrices may be rectangular or square and have between 4 and 20, preferably between 5 and 13, sensors 4 per row or column.

An exemplary setup has been described in Raul C. Simpetru et al.: "Proportional and Simultaneous Real-Time Control of the Full Human Hand From High-Density Electromyography", IEEE Transactions on neural systems and rehabilitation engineering, Vol. 31, 2023 (https://doi.org/10.1109/TNSRE.2023.3295060).

The sEMG signal may be pre-processed, e.g., filtered. In particular, a temporal and/or spatial domain filter may be employed. Also, the sensor signals may be simplified by omitting certain signals and/or calculating an average over neighbouring channels (ablation).

The temporal filtering may be performed on the original (raw) data. As a temporal filter, a

Butterworth filter may be used. The Butterworth filter may be of the 4th order having a cutoff frequency of 5 and 20 Hz, respectively. Other frequency values may be suitable depending on the use case.

Moreover, the raw signal may be normalised prior to filtering.

The spatial filter may comprise at least one of a longitudinal single differential filter (LSD), transversal single differential filter (TSD), longitudinal double differential filter (LDD), transversal double differential filter (TDD), inverse binomial filter of order two (IB2), or inverse rectangle filter (IR).

The filters may be characterised by their masks $\{K_x | x \in \{LSD, TSD, LDD, TDD, NDD, IB2, IR\}\}$ defined as follows.

$$K_{LSD} = \begin{bmatrix} +1 \\ -1 \end{bmatrix} \qquad K_{TSD} = \begin{bmatrix} +1 & -1 \end{bmatrix}$$

$$K_{LDD} = \begin{bmatrix} +1 \\ -2 \\ +1 \end{bmatrix} \qquad K_{TDD} = \begin{bmatrix} +1 & -2 & +1 \end{bmatrix}$$

$$K_{NDD} = \begin{bmatrix} 0 & -1 & 0 \\ -1 & 4 & -1 \\ 0 & -1 & 0 \end{bmatrix} \qquad K_{IB2} = \begin{bmatrix} -1 & -2 & -1 \\ -2 & 12 & -2 \\ -1 & -2 & -1 \end{bmatrix}$$

$$K_{IR} = \begin{bmatrix} -1 & -1 & -1 \\ -1 & 8 & -1 \\ -1 & -1 & -1 \end{bmatrix}$$

Also an ablation may be performed on the sensor signals. In particular, an average of a plurality of sensors 4 or sensor channels may be determined.

E.g., averages over a set of 5 neighbouring channels each, placed either close to or further away from each other, and computed the difference between them. This mimics the effect of a bipolar measurement that uses electrodes with a large surface area. In the above described scenario of a hand orthosis, the close placement may denote a distance of 15 - 25 mm, the farther placement may denote a distance of 45 - 55 mm between respective sensors 4. The distances may vary according to the placement of the sensors on a patient's body.

Alternatively, a randomised selection of sensors/electrodes 4 may be performed. The number of selected sensors 4 may be between 1% and 20% of the total number of electrodes. Also, to improve spreading of the signal, once a sensor 4 was picked, the probability of one of its neighbours to also be selected was lowered, which in turn increased the chance of sensors 4 from different areas of the grid to be chosen.

An artificial intelligence model may be used for processing the sensor data. Exemplary models are described in R. C. Sîmpetru, A. Arkudas, D. Braun, M. Osswald, D. Souza de Oliveira, B. Eskofier, T. M. Kinfe, and A. D. Vecchio, Sensing the full dynamics of the human hand with a neural interface and deep learning, Dec. 10, 2022. DOI: 10.1101/2022.07.29.502064, R. C. Sîmpetru, M. März, and A. Del Vecchio, "Proportional and simultaneous real-time control of the full human hand from high-density electromyography," IEEE Transactions on Neural Systems and Rehabilitation Engineering (also cited above) and R. C. Sîmpetru, M. Osswald, D. I. Braun, D. Souza de Oliveira, A. L. Cakici, and A. Del Vecchio, "Accurate continuous prediction of 14 degrees of freedom of the hand from myoelectrical signals through convolutive deep learning," in Proceedings of the 2022 44th Annual International Conference of the IEEE Engineering in Medicine & Biology Society (EMBC), Jul. 2022, pp. 702-706. DOI: 10/gq2f47.

The model's input may be a 3D array, where the width represents time, the height represents the number of electrodes, and the depth corresponds to different frequency filters. In the present embodiment, each frequency filter except for the Raw + 20 Hz filter may contain information specific to a particular frequency range resulting in a depth size of 1. The Raw + 20 Hz filter may combine the unfiltered data with the information filtered at 20 Hz using a lowpass filter and gives a depth of 2. The input may then be reshaped and standardised using gridwise min-max scaling.

As a deep learning model, a suitable model may be employed. In an embodiment, the deep learning model may consist of two parts, a convolutional neural network (CNN) and a multilayer perceptron (MLP). The first convolutional layer looks for overlaps in the motor unit action potentials such that patters can be found that are indicative of specific movements. These overlaps are then circular padded so the next layer can integrate not only inter-grid but also intra-grid information. The last CNN layer is used to further compress the information for the upcoming MLP. The second part of the model, the MLP, is used to map the high dimensional latent space produced by the CNN into 3D space.

Through the filtering or ablation process, complexity and required computational resources may be minimised. Thereby, it is possible to provide a small and efficient artificial intelligence model enabling data processing in real time. Moreover, the model can easily be parallelised. Each degree of freedom, in the case of a hand orthosis each digit or gesture, may comprise its own artificial intelligence model determining a user's intent in real time.

Low pass filtering the raw sensor data may, while lowering the complexity of the data, lower accuracy of the determination of a user's intent. However, low pass filtering rectified sEMG data may increase the accuracy of the determination of a user's intent. Moreover, reducing the number of electrodes by ablation as outlined above may improve the computational efficiency and lower the complexity but will also lead to a deterioration of the determination of a user's intent.

The processor 3 may be configured to train the artificial intelligence model based on the sEMG signal.

The training may be performed using an unsupervised approach. In an unsupervised approach according to an embodiment, artificial intelligence is used to automatically locate the bone in a first step. Bones are anisotropic, causing changes in the sign of motor unit action potentials measured at the skin surface. This change in sign help in identifying the location of the bone. By consistently placing the control system of the EMG measurement on the forearm in the same orientation, location changes may be detected based on equidistant interelectrode distances in electrode arrays.

As the artificial intelligence system according to the embodiment learns human physiology, it may interpret the measured HDsEMG signals in relation to the unique spatiotemporal features of the extrinsic muscles of the hand. The uniqueness of muscle and motor unit involvement in different movements allows the artificial intelligence to associate signals with specific hand actions. The ability to orient and adjust everything to a reference point, such as the bone, allows having an unsupervised artificial intelligence system that operates without user input. In a nutshell, the reconstruction of the dimensions of the EMG signals that correspond to the specific movement are performed by a newly integrated approach as outlined above that contains an algorithm to detect the bone location and another algorithm that separates the contribution of each muscle to a specific movement.

As outlined above, the assisting device 1 may be an orthosis, a prothesis or a robotic limb. Preferably, the orthosis is a hand orthosis. The assisting device 1 may be configured to independently move, via the actuators 2, the assisting device 1 in at least two degrees of freedom. In an embodiment, the number of degrees of freedom may be three. The degrees of freedom may be any digit or gesture. Hence, digits may be moved independently or certain movements such as fist, two finger pinch, three finger pinch, finger rotation, wrist rotation, etc. may be performed.

The assisting device 1 may further comprise at least one orientation sensor and/or at least one force sensor. This may further enhance the accuracy of the assisting device 1 and may also be used for training purposes.

At least one filter may be applied to the sEMG signal. As stated above, the filter may be a temporal filter, particularly a Butterworth filter and/or a spatial filter.

The system may comprise a plurality of sensors 4, wherein the processor 3 is configured to determine an average of the sEMG signals of at least two of the plurality of sensors 4.

The disclosure further relates to a method for training an artificial intelligence model for controlling an assisting device for supporting or moving a patient's body part.

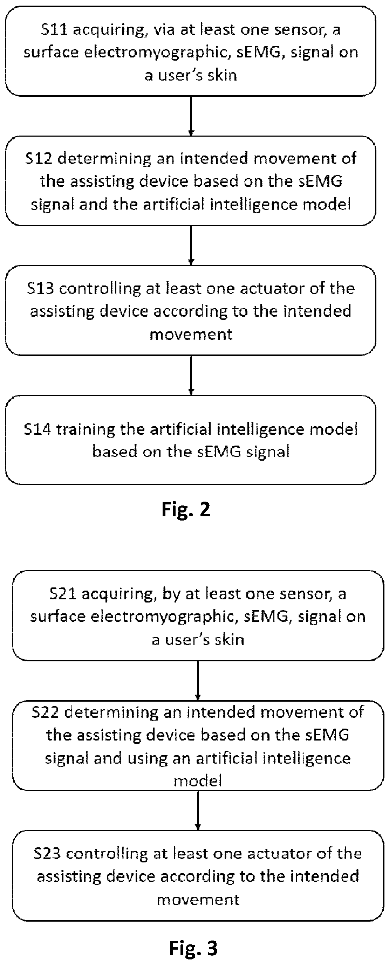

A flowchart of the method is depicted in Fig. 2 and the method according to this embodiment comprises acquiring S11, via at least one sensor, a surface electromyographic, sEMG, signal on a user's skin; determining S12 an intended movement of the assisting device based on the sEMG signal and the artificial intelligence model, and controlling S13 at least one actuator of the assisting device according to the intended movement. The method further comprises training S14 the artificial intelligence model based on the sEMG signal.

The training process may be performed with healthy, i.e., unimpaired subjects. The data collected during the training process may be used for impaired patients, wherein the artificial intelligence model is capable of adapting to the individual by continuous training. Alternatively, the training may be performed on the impaired patient him-/herself.

In an embodiment, the method further comprises applying at least one filter to the sEMG signal, wherein the filter preferably is a temporal filter, more preferably a Butterworth filter, and/or wherein the filter preferably is a spatial filter.

In an embodiment, the method further comprises determining an average of the sEMG signals of at least two of a plurality of sensors.

The present disclosure further relates to a corresponding method for controlling an assisting device for supporting or moving a patient's body part.

As shown in the flowchart of Fig. 3, the method comprises acquiring S21, by at least one sensor, a surface electromyographic, sEMG, signal on a user's skin, determining S22 an intended movement of the assisting device based on the sEMG signal and using an artificial intelligence model and controlling S23 at least one actuator of the assisting device according to the intended movement.

For the purpose of improving determination of a user's intent to move a body part, various factors and parameters have been taken into account.

For training purposes, movement of, e.g., the hand may be tracked by a camera. Thus, the intended and the actual movement may be compared and training of the artificial intelligence model can be accelerated and improved.

The system may further comprise a graphical user interface, GUI, allowing individualisation and adaptation of the (training) data and/or the artificial intelligence model. The GUI may be provided on a computer, a smartphone or the like which may be in wireless or wired communication to the processor 3.

When processing monopolar sEMG signals that were originally recorded from 320 electrodes over the forearm muscles of 13 subjects, the present disclosure used a deep learning method that can map the dynamics of the human hand with real-time resolution. While state-of-the-art myocontrol algorithms essentially use the temporal envelope of the EMG signal as the only EMG feature, in the presented approach, the full bandwidth of the signal in the temporal domain may be used for accurate estimates. Spatial filtering had a smaller impact and loworder spatial filters may be suitable. Moreover, reducing the number of channels by ablation resulted in large performance losses. The highest accuracy was reached with the highest number of available sensors (320). Moreover, increasing the number of channels above those used in this study may further enhance the accuracy in predicting the dynamics of the human hand.

The present disclosure utilises a non-invasive BMI system to control an assisting device. It may decode the neurophysiological electrophysiological signals from the muscles of a human subject to identify the motor dimensions that the subject can voluntarily control and those they cannot. As used herein, motor dimensions encompass the execution of specific movements, whether involving a single digit or a combination of at least two digit movements. Examples include actions like index flexion/extension or the opening and closing of a fist, each representing distinct motor dimensions.

The BMI system can decode as many motor dimensions as the subject consents to record and automatically assess their potential for rehabilitation and to what extent. The recorded neurophysiological data may be used to train an artificial intelligence (AI) system to predict the attempted movement. The AI may generate a high-dimensional internal representation of the data, which is then transformed into a human-readable 2D or 3D format and may be displayed through a graphical user interface. Additional algorithms can be configured to compute variables on the internal representations of the AI to enable the system to automatically identify successful attempted movements and assess their controllability by the subject. This step may help in order to understand in an automatic and intuitive way for the user the spared motor dimensions that are mapped into movements of the assistive devices.

With the presented methods and system, an improved control of an assisting device such as an orthosis, prothesis or robotic hand can be achieved. The disclosure reduces complexity and improves the computational efficiency enabling a real-time determination of a user's movement intent. Due to this increased efficiency, a separate artificial intelligence model for each degree of freedom may be used, if desired.

According to known training models, healthy subjects are targeted, whereas the presented artificial intelligence model extends its applicability to paralysed individuals and amputees.

Moreover, according to the present disclosure, a deep learning model for raw kinematics is presented, complemented by a latent space classification system to discern the intended gesture.

The presented training methodology differs from previous supervised approaches and includes non-supervised techniques. This novel strategy utilises the physiological characteristics of humans for more effective training and adaptation to changes and sets the present disclosure apart from previous known methodologies.

The system and method may thus be used to read injured muscle signals to automatically decode a patient's intent.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any one of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the network or within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein, but is to be accorded the widest scope consistent with the novel features and principles disclosed herein, as recited in the claims below.

**Claims**

1. System for controlling an assisting device (1) for supporting or moving a patient's body part, the system comprising:

    the assisting device (1);
    at least one actuator (2) connected to the assisting device (1);
    at least one processor (3);
    at least one sensor (4) configured to acquire a surface electromyographic, sEMG, signal on a user's skin;
    wherein the processor (3) is configured to determine an intended movement of the assisting device (1) based on the sEMG signal and using an artificial intelligence model, and
    wherein the processor (3) is configured to control the at least one actuator (2) according to the intended movement.

2. System according to claim 1,
    wherein the processor (3) is configured to train the artificial intelligence model based on the sEMG signal.

3. System according to claim 1 or 2, wherein the assisting device (1) is an orthosis, a prothesis or a robotic limb.

4. System according to any one of claims 1 to 3, wherein the orthosis is a hand orthosis.

5. System according to claim 4, wherein the assisting device (1) is configured to independently move, via a plurality of actuators (2), the assisting device (1) in at least two degrees of freedom.

6. System according to any one of claims 1 to 5, wherein the assisting device (1) further comprises at least one orientation sensor and/or at least one force sensor.

7. System according to any one of claims 1 to 6, wherein the processor (3) is further configured to apply at least one filter to the sEMG signal.

8. System according to claim 7, wherein the filter is a temporal filter, preferably a Butterworth filter.

9. System according to claim 7 or 8, wherein the filter is a spatial filter.

10. System according to any one of claims 1 to 9, wherein the system comprises a plurality of sensors (4),
    wherein the processor (3) is configured to determine an average of the sEMG signals of at least two of the plurality of sensors (4).

11. Method for training an artificial intelligence model for controlling an assisting device (1) for supporting or moving a patient's body part, the method comprising:

acquiring (S11), via at least one sensor (4), a surface electromyographic, sEMG, signal on a user's skin;
determining (S12) an intended movement of the assisting device (1) based on the sEMG signal and the artificial intelligence model, and
controlling (S13) at least one actuator (2) of the assisting device (1) according to the intended movement, and
training (S14) the artificial intelligence model based on the sEMG signal.

12. Method according to claim 11, further comprising applying at least one filter to the sEMG signal, wherein the filter preferably is a temporal filter, more preferably a Butterworth filter, and/or
wherein the filter preferably is a spatial filter.

13. Method according to any one of claims 11 or 12, further comprising determining an average of the sEMG signals of at least two of a plurality of sensors (4).

14. Method for controlling an assisting device (1) for supporting or moving a patient's body part, the method comprising:

acquiring (S21), by at least one sensor (4), a surface electromyographic, sEMG, signal on a user's skin;
determining (S22) an intended movement of the assisting device (1) based on the sEMG signal and using an artificial intelligence model, and
controlling (S23) at least one actuator (2) of the assisting device (1) according to the intended movement.

**Fig. 1**

S11 acquiring, via at least one sensor, a surface electromyographic, sEMG, signal on a user's skin

↓

S12 determining an intended movement of the assisting device based on the sEMG signal and the artificial intelligence model

↓

S13 controlling at least one actuator of the assisting device according to the intended movement

↓

S14 training the artificial intelligence model based on the sEMG signal

**Fig. 2**

S21 acquiring, by at least one sensor, a surface electromyographic, sEMG, signal on a user's skin

↓

S22 determining an intended movement of the assisting device based on the sEMG signal and using an artificial intelligence model

↓

S23 controlling at least one actuator of the assisting device according to the intended movement

**Fig. 3**

**EUROPEAN SEARCH REPORT**

Application Number

EP 24 16 8372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/343662 A1 (SONG AIGUO [CN] ET AL) 14 November 2019 (2019-11-14) * figures 1-4 * * paragraphs [0001], [0025] - [0059] * | 1-4,6-8, 11,12,14 | INV. A61F2/58 A61F2/72 A61F5/01 B25J9/00 A61F2/70 |
| X | EP 3 082 591 B1 (INTEGRUM AB [SE]) 23 August 2023 (2023-08-23) * figures 4-6 * * paragraphs [0008] - [0017], [0023] - [0031], [0044] - [0060], [0064] * | 1,3-7,9, 10,13,14 | |
| X | WO 2021/178914 A1 (NORTHWELL HEALTH INC [US]) 10 September 2021 (2021-09-10) * figure 9 * * paragraphs [0001], [0014], [0015], [0042], [0049], [0059] - [0061] * | 1-6,11, 14 | |
| A | SÎMPETRU RAUL C ET AL: "Influence of spatio-temporal filtering on hand kinematics estimation from high-density EMG signals**This work was supported by the German Federal Ministry of Education and Research (BMBF) through the project MYOREHAB under Grant 01DN23002, by the Bavarian Ministry of Economic Affairs, Regional Devel", JOURNAL OF NEURAL ENGINEERING, IOP PUBLISHING, BRISTOL, GB, vol. 21, no. 2, 25 March 2024 (2024-03-25) , XP020495956, ISSN: 1741-2560, DOI: 10.1088/1741-2552/AD3498 [retrieved on 2024-03-25] * the whole document * | 1-14 | |

TECHNICAL FIELDS SEARCHED (IPC)

A61F
B25J

The present search report has been drawn up for all claims

| Place of search | Date of completion of the search | Examiner |
|---|---|---|
| Munich | 8 August 2024 | Harnack, Hanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 8372

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2024

| Patent document cited in search report | | Publication date | Patent family member(s) | | Publication date |
|---|---|---|---|---|---|
| US 2019343662 | A1 | 14-11-2019 | CN | 107378944 A | 24-11-2017 |
| | | | US | 2019343662 A1 | 14-11-2019 |
| | | | WO | 2018233435 A1 | 27-12-2018 |
| EP 3082591 | B1 | 23-08-2023 | AU | 2014367277 A1 | 09-06-2016 |
| | | | CA | 2933053 A1 | 25-06-2015 |
| | | | EP | 3082591 A1 | 26-10-2016 |
| | | | US | 2017025026 A1 | 26-01-2017 |
| | | | US | 2018082600 A1 | 22-03-2018 |
| | | | US | 2023058936 A1 | 23-02-2023 |
| | | | WO | 2015094112 A1 | 25-06-2015 |
| WO 2021178914 | A1 | 10-09-2021 | AU | 2021231896 A1 | 22-09-2022 |
| | | | CA | 3170484 A1 | 10-09-2021 |
| | | | CN | 115279453 A | 01-11-2022 |
| | | | EP | 4114505 A1 | 11-01-2023 |
| | | | JP | 2023516309 A | 19-04-2023 |
| | | | US | 2021275807 A1 | 09-09-2021 |
| | | | WO | 2021178914 A1 | 10-09-2021 |

EPO FORM P0459

For more details about this annex : see Official Journal of the European Patent Office, No. 12/82

## REFERENCES CITED IN THE DESCRIPTION

**Non-patent literature cited in the description**

- **RAUL C. SIMPETRU et al.** Proportional and Simultaneous Real-Time Control of the Full Human Hand From High-Density Electromyography. *IEEE Transactions on neural systems and rehabilitation engineering*, 2023, vol. 31, https://doi.org/10.1109/TNSRE.2023.3295060
- **R. C. SÎMPETRU** ; **A. ARKUDAS** ; **D. BRAUN** ; **M. OSSWALD** ; **D. SOUZA DE OLIVEIRA** ; **B. ESKOFIER** ; **T. M. KINFE** ; **A. D. VECCHIO**. *Sensing the full dynamics of the human hand with a neural interface and deep learning*, 10 December 2022
- **R. C. SÎMPETRU** ; **M. MÄRZ** ; **A. DEL VECCHIO**. Proportional and simultaneous real-time control of the full human hand from high-density electromyography. *IEEE Transactions on Neural Systems and Rehabilitation Engineering*
- **R. C. SÎMPETRU** ; **M. OSSWALD** ; **D. I. BRAUN** ; **D. SOUZA DE OLIVEIRA** ; **A. L. CAKICI** ; **A. DEL VECCHIO**. Accurate continuous prediction of 14 degrees of freedom of the hand from myoelectrical signals through convolutive deep learning. *Proceedings of the 2022 44th Annual International Conference of the IEEE Engineering in Medicine & Biology Society (EMBC)*, July 2022, 702-706